# EUROPÄISCHE PATENTSCHRIFT

(11) **EP 0 655 441 B1**
(45) Veröffentlichungstag und Bekanntmachung des Hinweises auf die Patenterteilung: **23.01.2002**
(21) Anmeldenummer: 94810626.5
(22) Anmeldetag: 01.11.1994
(51) Int. Cl.: C07D 239/42, A01N 43/54

(54) **Kristallmodifikation von (4-Cyclopropyl-6-methyl-pyrimidin-2-yl)- phenyl-amin, und Verfahren zu dessen Herstellung**
Crystalline modification of (4-cyclopropyl-6-methyl-pyrimidin-2-yl)-phenyl-amine, and process for its preparation
Modification cristalline de (4-cyclopropyle-6-méthyle-pyrimidin-2-yle)-phényl-amine, et procédé de son préparation

(30) Priorität: 09.11.1993 CH 336893; 28.07.1994 CH 239394
(43) Veröffentlichungstag der Anmeldung: 31.05.1995
(73) Patentinhaber: Syngenta Participations AG, 4058 Basel (CH)
(72) Erfinder: Baettig, Willy, CH-4133 Pratteln (CH); Hanreich, Reinhard Georg, Dr., CH-4055 Basel (CH)

(56) Entgegenhaltungen:
- EP-A- 0 310 550
- PHYTOMA, Nr.458, Februar 1994 Seiten 53 - 55 BOCQUET G. ET AL. 'Le Cyprodinil. Fongicide céréales'
- CROP PROTECTION, Bd.13, Nr.7, Juli 1994 Seiten 541 - 549 HEYE U.J. ET AL. 'CGA 219417: a novel broad-spectrum fungicide'
- PESTICIDE SCIENCE, Bd.42, Nr.3, 1994 Seiten 163 - 166 MASNER P. ET AL. 'Possible methionine biosynthesis inhibition by pyrimidinamine fungicides'
- MONZEL K.: 'Der Einfluss der Formgebung auf die Wirkung eines Arzneimittels' FORTSCHRITTE DER ARZNEIMITTELFORSCHUNG Bd. 10, 1966, BIRKHOUSER VERLAG, BASEL, Seiten 227 - 230

## Beschreibung

Die vorliegende Erfindung betrifft (4-Cyclopropyl-6-methyl-pyrimidin-2-yl)-phenyl-amin der Kristallmodifikation B mit einem Schmelzpunkt oberhalb 73°C, bevorzugt 73 - 75°C, ein Verfahren zur Herstellung dieser Kristallmodifikation, ein Mittel, das diese Kristallmodifikation enthält, und seine Verwendung zur Bekämpfung von Pilzbefall in Pflanzenkulturen.

Aus der EP-A-0,310,550 ist (4-Cyclopropyl-6-methyl-pyrimidin-2-yl)-phenyl-amin der Kristallmodifikation A mit einem Schmelzpunkt zwischen 67°C und 69°C bekannt. Das Fungizid wirkt gegen eine Reihe von Krankheiten, die durch Ascomyceten oder Deuteromyceten verursacht werden. Feste Formulierungen dieser Wirksubstanz zeigen jedoch nur eine geringe Lagerstabilität, die sich besonders durch ein unerwünschtes Kristallwachstum äussert. In der Praxis führt dies beispielsweise dazu, dass die für die Applikation hergestellte Spritzbrühe ein ungenügendes Suspendier- bzw. Dispergierverhalten aufweist und in der Folge die Spritzdüsen verstopft.

Es wurde nun überraschend gefunden, dass durch geeignete Wahl des Kristallisationsprozesses für (4-Cyclopropyl-6-methyl-pyrimidin-2-yl)-phenyl-amin eine neue Kristallmodifikation B hergestellt werden kann, die diese unerwünschten Eigenschaften nicht aufweist. Die neue Kristallmodifikation B besitzt einen Schmelzpunkt zwischen 73°C und 75°C und unterscheidet sich sowohl im Röntgenpulverdiagramm (siehe Tabelle 1) als auch im IR-Spektrum von der tiefer schmelzenden Kristallmodifikation A (siehe abgebildete IR-Spektren 1.1 und 1.2).
Die erfindungsgemässe Kristallmodifikation B weicht somit durch ihren Schmelzpunkt, ihr IR-Spektrum und das Röntgenpulverdiagramm in charakteristischer Weise von der Modifikation A ab.

**Tabelle 1 :**

| Röntgenpulverdiagramm. Aufnahme mit einer Guinier-Kamera (FR 552 von Enraf-Nonius) in Durchstrahlungsgeometrie mit Quarz als internem Standard und unter Verwendung von Kupfer-Kα₁-Strahlung (λ = 1.54060Å) auf Röntgenfilm. | | | |
|---|---|---|---|
| Kristallmodifikation A | | Kristallmodifikation B | |
| d-Wert (Å) | Intensität | d-Wert (Å) | Intensität |
| 13.0 | mittel | 12.9 | mittel |
| 7.8 | mittel | 8.7 | stark |
| 6.6 | mittel | 6.8 | stark |
| 6.5 | schwach | 6.1 | schwach |
| 5.74 | sehr schwach | 5.93 | sehr schwach |
| 5.06 | sehr stark | 5.66 | stark |
| 4.90 | schwach | 5.39 | schwach |
| 4.81 | stark | 5.19 | sehr schwach |
| 4.49 | sehr schwach | 4.96 | schwach |
| 4.39 | schwach | 4.81 | mittel |
| 4.11 | mittel | 4.75 | mittel |
| 3.93 | mittel | 4.55 | sehr stark |
| 3.89 | stark | 4.47 | mittel |
| 3.60 | schwach | 4.36 | schwach |
| 3.54 | sehr stark | 3.97 | schwach |
| 3.34 | stark | 3.86 | mittel |
| 3.30 | schwach | 3.80 | sehr stark |
| 3.22 | sehr schwach | 3.78 | mittel |
| 3.16 | schwach | 3.67 | mittel |
| 3.12 | sehr schwach | 3.56 | mittel |
| | | 3.54 | sehr schwach |
| | | 3.42 | mittel |
| | | 3.38 | schwach |
| | | 3.30 | mittel |
| | | 3.25 | sehr schwach |
| | | 3.16 | schwach |
| | | 3.09 | schwach |
| | | 3.04 | sehr schwach |

Feste Formulierungen mit der neuen Kristallmodifikation B haben gegenüber Formulierungen mit der bekannten Modifikation A den eindeutigen Vorteil, dass sie eine hohe Lagerstabilität aufweisen und nach langen Lagerzeiten sogar bei erhöhten Temperaturen ihre vorzüglichen physikochemischen Eigenschaften wie Suspendier- und Dispergierbarkeit behalten.

Thermodynamische Untersuchungen haben gezeigt, dass sich kristallines (4-Cyclopropyl-6-methyl-pyrimidin-2-yl)-phenyl-amin der Kristallmodifikation A in Gegenwart eines Lösungsvermittlers (z.Bsp. organisches Lösungsmittel wie Toluol oder Methylcyclohexan) bei einer Temperatur um 26°C innerhalb weniger Stunden vollständig in die neue Kristallmodifikation B überführen lässt. Unterhalb dieser Temperatur findet, wenn auch nach wesentlich längerer Zeitdauer, die quantitative Umwandlung der Kristallmodifikation B nach A statt. Dieser Umwandlungsprozess spielt bei der agrochemischen Anwendung jedoch keine Rolle.

Unter Ausschluss eines Lösungsvermittlers lässt sich die Kristallmodifikation A bei einer Temperatur knapp unterhalb des Schmelzpunktes von 67 - 69°C in die höher schmelzende Modifikation B überführen. Dieser Vorgang kann besonders während des Mahlvorganges in einer mechanischen Mühle beobachtet werden.
Überraschend ist hingegen die Feststellung, dass eine Modifikationsumwandlung von B nach A in Abwesenheit eines Lösungsvermittlers nicht nachgewiesen werden kann, was für die Praxis von grösster Bedeutung ist. Somit werden lagerstabile Formulierungen der Modifikation B geschaffen, die sich auch bei tieferen Temperaturen, z.B. in der Nähe des Gefrierpunkts, nicht in solche der Modifikation A umwandeln. Versuche mit gesättigten Lösungen der beiden Modifikationen haben folgende Zusammenhänge ergeben:

Versuchsbedingungen : Herstellung einer gesättigten Lösung von (4-Cyclopropyl-6-methyl-pyrimidin-2-yl)-phenyl-amin in Toluol. Anschliessend 2-3 Stunden Rühren und mit ca. 20 mg Substanz Animpfen. Weitere 2-3 Stunden Rühren und danach den Festkörper abfiltrieren. Das Kristallisat bei der entsprechenden Temperatur am Vakuum trocknen. Modifikationsbestimmung des trockenen Kristallisates mittels DSC-Messung (Schmelzpunkt).

| Temperatur [°C] | Ausgangsbedingung: gesättigte Lösung mit Bodenkörper der **Kristallmodifikation A** | Ausgangsbedingung: gesättigte Lösung mit Bodenkörper der **Kristallmodifikation B** |
|---|---|---|
| 20 | A + Animpfen mit A →A | B + Animpfen mit A →A |
| | A + Animpfen mit B →A | B + Animpfen mit B →A |
| 26 | A + Animpfen mit A →A | B + Animpfen mit A →A/B |
| 30 | A + Animpfen mit B →A | B + Animpfen mit A →B |
| 35 | A + Animpfen mit B →B | B + Animpfen mit A →B |

Für die Praxisanwendung ist daher das Vorliegen eines möglichst hohen Anteils an Modifikation B wichtig, um weitere Kristallumwandlungen A ---> B bei der Lagerung oder der Anwendung (Verstopfen von Spritzdüsen bzw. Verklumpen der formulierten Ware) zu vermeiden.

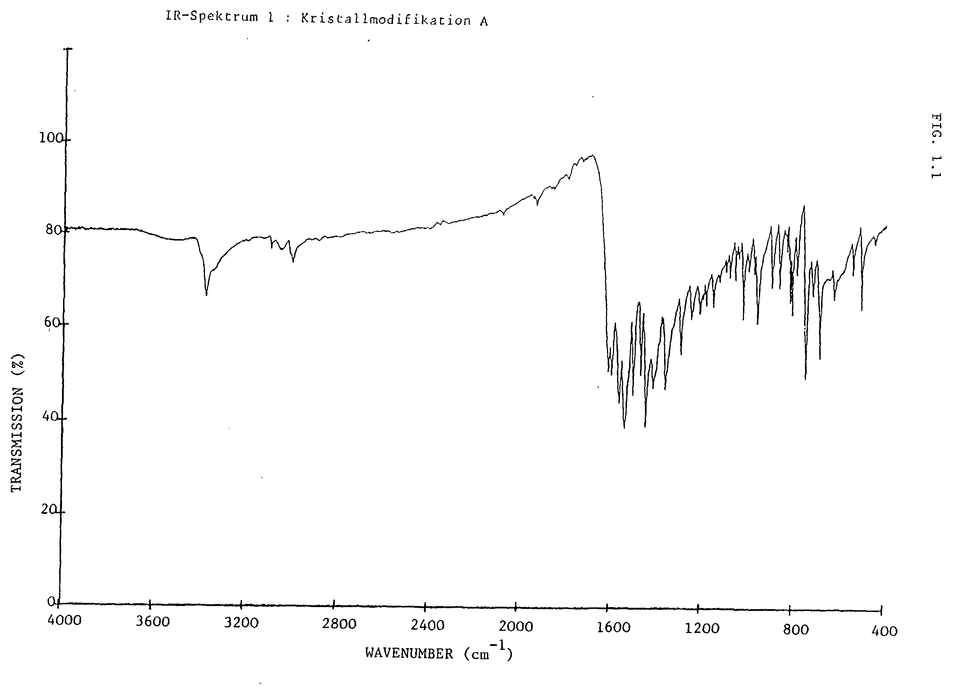

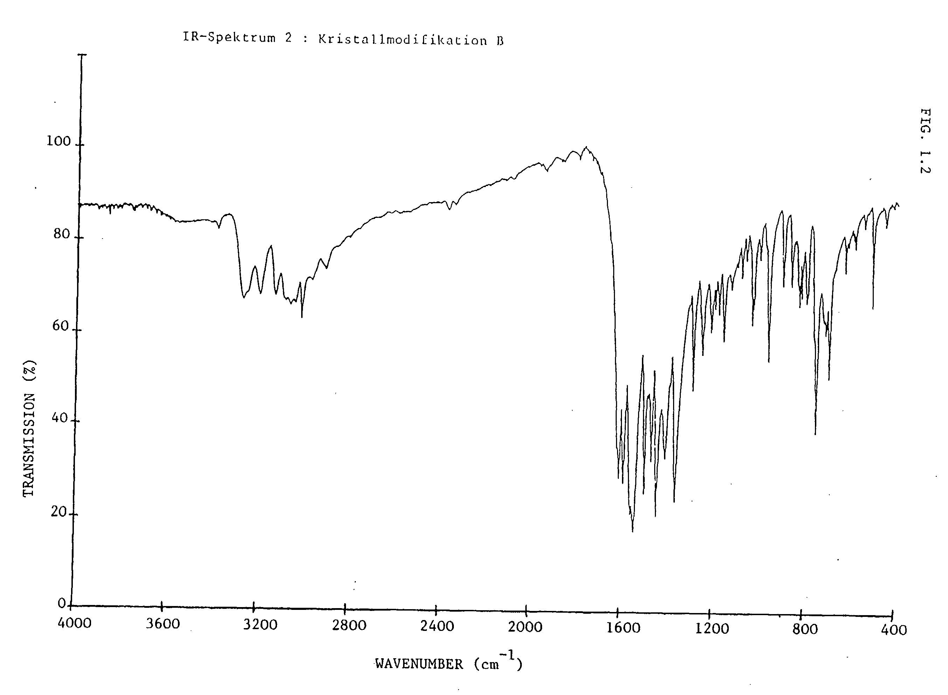

Ein Gegenstand der vorliegenden Erfindung ist das (4-Cyclopropyl-6-methyl-pyrimidin-2-yl)-phenyl-amin der Kristallmodifikation B, in hoher eutektischer Reinheit (Gehalt mindestens 98%) mit einem Schmelzpunkt grösser als 73°C, bevorzugt 73°C - 75°C, einem IR-Spektrum gemäss Fig. 1.2 mit einer charakteristischen NH-Bande bei 3200-3300 cm⁻¹ (st = Streckschwingung) und einem Röntgenpulverdiagramm unter Verwendung der Kupfer-Kα₁-Strahlung mit den Daten gemäss Tabelle 1.
Es ist zu beachten, dass schnelles Aufheizen einer Stoffprobe der Modifikation B zu einem scheinbaren Schmelzpunkt von 74.5 bis 76°C führen kann. Es handelt sich indessen um die gleiche kristalline B-Modifikation.

Ein weiterer Gegenstand der vorliegenden Erfindung ist ein Verfahren zur wirtschaftlichen Herstellung von (4-Cyclopropyl-6-methyl-pyrimidin-2-yl)-phenyl-amin in der Kristallmodifikation B, dadurch gekennzeichnet, dass diese durch Schmelzkristallisation hergestellt wird.

Chemische Verfahren zur Herstellung von (4-Cyclopropyl-6-methyl-pyrimidin-2-yl-)-phenyl-amin sind in der EP-A-0,310,550 beschrieben. Für die neue Kristallmodifikation B hingegen wird die Wirksubstanz aus einem geeigneten Lösungsmittel (z.Bsp. i-Propanol, Methylcyclohexan) kristallisiert oder durch Abdestillieren des Lösungsmittels als Rohschmelze gewonnen. Um die notwendige Reinheit zu erzielen, wird diese anschliessend über einen Dünnschichtverdampfer destilliert. Beide Qualitäten der Wirksubstanz (aus dem Kristallisationsverfahren bzw. Schmelzeverfahren) sind geeignet, um nach dem Schmelzkristallisationsprozess die gewünschte Kristallmodifikation B in hoher eutektischer Reinheit zu liefern. Dabei wird die heisse Produktschmelze in einer geeigneten Vorrichtung auf 72 - 75°C, vorzugsweise 74°C abgekühlt. In einer besonderen Ausgestaltung des Verfahrens werden dabei die sich bildenden Kristalle von der gekühlten Kesselwand abgeschabt. Eine Temperatur der Kesselwand von 40°C bis 60°C, insbesondere von 50°C hat sich als sehr günstig erwiesen. Die so erhaltene mit Kristallkeimen versehene Schmelze wird zur Vervollständigung des Kristallisationsprozesses weiter abgekühlt. Vorteilhafterweise wird diese Schmelze über eine geeignete Vorrichtung auf eine gekühlte Fläche (z.B. Schuppierwalze oder Schuppierband) geleitet, bis die Kristallisation abgeschlossen ist.

Ein weiterer Gegenstand der vorliegenden Erfindung ist eine Zusammensetzung, die als Aktivsubstanz (4-Cyclopropyl-6-methyl-pyrimidin-2-yl-)-phenyl-amin der Kristallmodifikation B mit einem Schmelzpunkt oberhalb 73°C, bevorzugt 73 - 75°C, und einen geeigneten Träger enthält. In einer besonderen Ausgestaltung der vorliegenden Erfindung kann die Zusammensetzung auch weitere Fungizide, Bakterizide, selektive Herbizide sowie Insektizide, Nematizide, Molluskizide oder Gemische mehrerer dieser Wirkstoffe enthalten. Solche fungiziden Zusammensetzungen oder Mittel stellen einen weiteren Gegenstand der Erfindung dar.

Darüber hinaus schliesst die vorliegende Erfindung auch die Herstellung dieser Mittel ein, die gekennzeichnet ist durch das innige Vermischen der Aktivsubstanz mit einem oder mehreren Trägern und gegebenenfalls einem anderen Wirkstoff. Eingeschlossen ist auch ein Verfahren zur Behandlung von Pflanzen, das sich durch die Applikation einer fungizid wirksamen Menge (4-Cyclopropyl-6-methyl-pyrimidin-2-yl-)-phenyl-amin der Kristallmodifikation B bzw. der neuen Zusammensetzung auszeichnet.

(4-Cyclopropyl-6-methyl-pyrimidin-2-yl-)-phenyl-amin der Kristallmodifikation B kann in unveränderter Form, d.h. wie es bei der Herstellung anfällt, eingesetzt werden, vorzugsweise verarbeitet man es aber auf übliche Weise mit den in der Formulierungstechnik gebräuchlichen Hilfsmitteln z.B. Suspensionen, Spritzpulvern, löslichen Pulvern, Stäubemitteln, Granulaten oder Mikrokapseln. Die Anwendungsverfahren wie Versprühen, Vernebeln, Verstäuben, Benetzen, Verstreuen oder Gießen werden gleich wie die Art der Mittel den angestrebten Zielen und den gegebenen Verhältnissen entsprechend gewählt.

Die Formulierungen, d.h. die (4-Cyclopropyl-6-methyl-pyrimidin-2-yl-)-phenyl-amin der Kristallmodifikation B mit einem Schmelzpunkt zwischen 73°C und 75°C enthaltenden Mittel, Zubereitungen oder Zusammensetzungen können in bekannter Weise hergestellt werden, z.B. durch inniges Vermischen und/oder Vermahlen der Wirkstoffe mit dem Träger oder den Trägern.

Träger im Rahmen der vorliegenden Erfindung können sowohl fest als auch flüssig sein. Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Beispiele für flüssige Träger sind Lösungsmittel und oberflächenaktive Verbindungen (Tenside).

Als Lösungsmittel können in Frage kommen: Aromatische Kohlenwasserstoffe, insbesondere die Fraktionen C₈ bis C₁₂, wie Mischungen von Alkylbenzolen, z.B. Xylolgemische oder alkylierte Naphthaline; aliphatische und cycloaliphatische Kohlenwasserstoffe wie Paraffine, Cyclohexan oder Tetrahydronaphthalin; Alkohole, wie Ethanol, Propanol oder Butanol; Glykole sowie deren Ether und Ester, wie Propylenglykol oder Dipropylenglykolether, Ketone wie Cyclohexanon, Isophoron oder Diacetonalkohol, starke polare Lösungsmittel wie N-Methyl-2-pyrrolidon, Dimethylsulfoxid oder Wasser; Pflanzenöle sowie deren Ester, wie Raps-, Ricinus- oder Sojaöl; gegebenenfalls auch Silikonöle.

Als oberflächenaktive Verbindungen kommen nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali oder gegebenenfalls substituierten Ammoniumsalze von höheren Fettsäuren (C₁₀-C₂₂), wie z.B. die Na- oder K-Salze der Öl- oder Stearinsäure, oder von natürlichen Fettsäuregemischen genannt, die z.B. aus Kokosnuß- oder Talgöl gewonnen werden können. Ferner sind auch die Fettsäure-methyl-taurinsalze zu erwähnen.

Häufiger werden jedoch sog. synthetische Tenside verwendet, insbesondere Fettalkoholsulfonate, Fettalkoholsulfate, sulfonierte Benzimidazolderivate oder Alkylarylsulfonate.

Die Fettalkoholsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschließt, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen, enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht ionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die in der Formulierungstechnik gebräuchlichen Tenside, die auch in den erfindungsgemäßen Mitteln verwendet werden können, sind u.a. in folgenden Publikationen beschrieben:
- "Mc Cutcheon's Detergents and Emulsifiers Annual", Mc Publishing Corp., Glen Rock, New Jersey, 1988.
- M. and J. Ash, "Encyclopedia of Surfactants", Vol. I-III, Chemical Publishing Co., New York, 1980-1981.
- Dr. Helmut Stache "Tensid-Taschenbuch", Carl Hanser Verlag, München/Wien 1981.

Die fungiziden Zubereitungen enthalten in der Regel 0,1 bis 99 % (w/w), insbesondere 0,1 bis 95 % (w/w), (4-Cyclopropyl-6-methyl-pyrimidin-2-yl-)-phenyl-amin der Kristallmodifikation B, 1 bis 99 % (w/w) eines festen oder flüssigen Zusatzstoffes und 0 bis 25 % (w/w), insbesondere 0,1 bis 25 % (w/w) eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe zur Erzielung spezieller Effekte enthalten. Diese weiteren Wirkstoffe können Spurenelemente-Vermittler oder andere das Pflanzenwachstum beeinflussende Präparate sein.

Insbesondere setzen sich bevorzugte Formulierungen folgendermaßen zusammen: (% = Gewichtsprozent)

| Stäube: | |
|---|---|
| Aktiver Wirkstoff | 0,1 bis 50 %, vorzugsweise 0,1 bis 1 % |
| festes Trägermittel | 99,9 bis 90 %, vorzugsweise 99,9 bis 99 % |

| Suspensions-Konzentrate: | |
|---|---|
| Aktiver Wirkstoff | 5 bis 75 %, vorzugsweise 10 bis 50 % |
| Wasser | 94 bis 24 %, vorzugsweise 88 bis 30 % |
| oberflächenaktives Mittel | 1 bis 40 %, vorzugsweise 2 bis 30 % |

| Benetzbare Pulver: | |
|---|---|
| Aktiver Wirkstoff | 0,5 bis 90 %, vorzugsweise 1 bis 80 % |
| oberflächenaktives Mittel | 0,5 bis 20 %, vorzugsweise 1 bis 15 % |
| festes Trägermaterial | 5 bis 95 %, vorzugsweise 15 bis 90 % |

| Granulate: | |
|---|---|
| Aktiver Wirkstoff | 0,1 bis 30 %, vorzugsweise 0,1 bis 15 % |
| festes Trägermittel | 99,5 bis 70 %, vorzugsweise 97 bis 85 % |

Das erfindungsgemässe (4-Cyclopropyl-6-methyl-pyrimidin-2-yl-)-phenyl-amin wird in der Regel mit Aufwandmengen von 0,001 bis 2 kg/ha insbesondere 0,005 bis 1 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und dem Befallsdruck der Krankheit sowie von der Applikation (Ort, Zeit, Verfahren) und kann, bedingt durch diese Parameter, innerhalb weiter Bereiche variieren.

Das erfindungsgemässe (4-Cyclopropyl-6-methyl-pyrimidin-2-yl-)-phenyl-amin in der Kristallmodifikation B wird üblicherweise in Form von Zusammensetzungen verwendet und kann gleichzeitig oder nacheinander mit weiteren Wirkstoffen auf die zu behandelnde Fläche oder Pflanze gegeben werden.

Ein bevorzugtes Verfahren zum Aufbringen des erfindungsgemässen (4-Cyclopropyl-6-methyl-pyrimidin-2-yl-)-phenyl-amin in der Kristallmodifikation B bzw. eines agrochemischen Mittels, das diese Wirksubstanz enthält, ist das Aufbringen auf das Blattwerk (Blattapplikation). Applikationsfrequenz und Aufwandmenge richten sich dabei nach dem Befallsdruck des betreffenden Erregers. Die erfindungsgemässe Kristallmodifikation B kann aber auch über den Erdboden durch das Wurzelwerk in die Pflanze gelangen (systemische Wirkung), indem man den Standort der Pflanze mit einer flüssigen Zubereitung tränkt oder die Substanz in fester Form in den Boden einbringt, z.B. in Form von Granulat (Bodenapplikation). Bei Wasserreiskulturen kann man solche Granulate dem überfluteten Reis-Feld zudosieren. Das erfindungsgemässe (4-Cyclopropyl-6-methyl-pyrimidin-2-yl-)-phenyl-amin in der Kristallmodifikation B kann aber auch auf Samenkörner (= Saatgut) aufgebracht werden (Coating), indem man die Körner entweder in einer flüssigen Zubereitung tränkt oder sie mit einer festen Zubereitung beschichtet. Ein weiteres vorteilhaftes Verfahren der Applikation ist die kontrollierte Wirkstoffabgabe. Dazu wird der Wirkstoff in Lösung auf mineralische Granulatträger oder polymerisierte Granulate (Hamstoff/Formaldehyd) aufgezogen und trocknen gelassen. Gegebenenfalls kann zusätzlich ein Überzug aufgebracht werden (Umhüllungsgranulate), der es erlaubt, den Wirkstoff über einen bestimmten Zeitraum dosiert abzugeben. Das Granulat wird dann in bekannter Weise ausgebracht.

Das erfindungsgemässe (4-Cyclopropyl-6-methyl-pyrimidin-2-yl-)-phenyl-amin in der Kristallmodifikation B besitzt ein für praktische Bedürfnisse sehr günstiges biozides Spektrum zur Bekämpfung von Pilzbefall. Es besitzt sehr vorteilhafte kurative, präventive und insbesondere systemische Eigenschaften und wird zum Schutz von zahlreichen Kulturpflanzen eingesetzt. Mit ihm kann an Pflanzen oder Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile z.B. vor phytopathogenen Mikroorganismen verschont bleiben.

Das erfindungsgemässe (4-Cyclopropyl-6-methyl-pyrimidin,2-yl-)-phenyl-amin ist beispielsweise gegen die den folgenden Klassen angehörenden phytopathogenen Pilze wirksam: Fungi imperfecti (insbesondere *Botrytis,* ferner *Pyricularia, Helminthosporium, Fusarium, Septoria, Cercospora* und *Altemaria); Basidiomyceten (z.B. Rhizoctonia, Hemileia, Puccinia).* Darüber hinaus wirkt es gegen die Klasse der *Ascomyceten (*z.B. *Venturia* und *Erysiphe, Podosphaera, Monilinia, Uncinula)* und der *Oomyceten (*z.B. *Phytophthora, Pythium, Plasmopara)*.
(4-Cyclopropyl-6-methyl-pyrimidin-2-yl-)-phenyl-amin in der Kristallmodifikation B kann ferner als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden. Es ist darüberhinaus gegen Schadinsekten wirksam, z.B. gegen Getreide-Schädlinge, insbesondere Reisschädlinge.

Als Zielkulturen für den hierin offenbarten pflanzenschützenden Einsatz gelten im Rahmen dieser Erfindung beispielsweise folgende Pflanzenarten: Getreide (Weizen, Gerste, Roggen, Hafer, Reis, Mais, Sorghum und verwandte Species); Rüben (Zucker- und Futterrüben); Kern-, Stein und Beerenobst (Äpfel, Birnen, Pflaumen, Pfirsiche, Mandeln, Kirschen, Erd-, Him- und Brombeeren); Hülsenfrüchte (Bohnen, Linsen, Erbsen, Soja); Ölkulturen (Raps, Senf, Mohn, Oliven, Sonnenblumen, Kokos, Rizinus, Kakao, Erdnüsse); Gurkengewächse (Kürbis, Gurken, Melonen); Fasergewächse (Baumwolle, Flachs, Hanf, Jute); Citrusfrüchte (Orangen, Zitronen, Pampelmusen, Mandarinen); Gemüsesorten (Spinat, Kopfsalat, Spargel, Kohlarten, Möhren, Zwiebeln, Tomaten, Kartoffeln, Paprika); Lorbeergewächse (Avocado, Cinnamonium, Kampfer) oder Pflanzen wie Tabak, Nüsse, Kaffee, Zuckerrohr, Tee, Pfeffer, Weinreben, Hopfen, Bananen- und Naturkautschukgewächse sowie Zierpflanzen.

Die nachfolgenden Beispiele erläutern die Erfindung näher.

### Herstellungsbeispiele

### Beispiel H1: Herstellung von (4-Cyclopropyl-6-methyl-pyrimidin-2-yl-)-phenyl-amin

90 kg Phenylguanidin-carbonat werden in 190 kg Methylcyclohexan suspendiert und mit 63.3 kg 1-Cyclopropyl-1,3-butandion versetzt. Anschliessend wird unter Rühren während 6 Stunden bei 100 - 110°C das sich bildende Reaktionswasser azeotrop abdestilliert. Nach dem Abkühlen auf 50 - 60°C wird mit 80 kg Wasser bei pH 3-4 extrahiert und die wässrige Phase abgetrennt. Auf Zugabe von 50 kg Wasser wird bei pH 9-10 ein zweites Mal extrahiert. Die wässrige Phase wird erneut abgetrennt und die organische Phase zum Rückfluss auf 105 - 110°C aufgeheizt, um Restwasser azeotrop zu entfernen.
Die Isolierung des Produktes erfolgt entweder A) als Schmelze oder B) durch Kristallisation.
A) Soll (4-Cyclopropyl-6-methyl-pyrimidin-2-yl)-phenyl-amin als Schmelze isoliert werden, wird das Lösungsmittel z.B. über einen Fallfilmverdampfer unter reduziertem Druck vollständig abdestilliert. In einer anschliessenden zweiten Druckstufe wird das Produkt über einen Dünnschichtverdampfer destilliert und danach als heisse Schmelze dem Schmelzkristallisationsprozess zugeführt.
B) Zur Kristallisation des Produktes wird die organische Lösung auf 37-40°C abgekühlt, bis die Kristallisation einsetzt. Danach wird weiter abgekühlt und anschliessend das Produkt abfiltriert. Der feuchte Nutschkuchen wird mit 80 kg Methylcyclohexan gewaschen und im Vakuum bei 45-50°C getrocknet. Das getrocknete Produkt kann dann, sofern gewünscht, auch geschmolzen und ebenfalls der Schmelzkristallisation zugeführt werden.

### Beispiel H2: Herstellung von (4-Cyclopropyl-6-methyl-pyrimidin-2-yl)-phenyl-amin der Kristallmodifikation B mit einem Schmelzpunkt von 73 - 75°C

Die heisse, kontinuierlich eingespeiste Produktschmelze wird in einem Kratzkessel (Volumen: 250 Liter, Füllgrad: 75%) abgekühlt und bei 74°C gehalten. Durch einen speziellen rotierenden Rührarm, der nahe an der auf 50°C gekühlten Kesselwand vorbeiführt, werden die sich bildenden Kristalle von der Kesselwand abgeschabt. Die so erhaltene mit Kristallkeimen versehene Schmelze wird kontinuierlich dem Kessel entnommen und über eine geeignete Verteilvorrichtung auf eine gekühlte Fläche zur Formgebung als Schuppen, Pillen usw. gefördert. Nach Abschluss des Kristallisationsvorganges wird (4-Cyclopropyl-6-methyl-pyrimidin-2-yl)-phenyl-amin als Kristallmodifikation B in den Formulierungsprozess eingeschleust.

### Formulierungsbeispiele

### Beispiel F1: Spritzpulver

| | a) | b) | c) |
|---|---|---|---|
| Wirkstoff in Kristallmodifikation B | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält ein Spritzpulver, das sich mit Wasser zu Suspensionen jeder gewünschten Konzentration verdünnen lässt.

### Beispiel F2: Stäubemittel

| | a) | b) |
|---|---|---|
| Wirkstoff in Kristallmodifikation B | 5 % | 8 % |
| Talkum | 95 % | - |
| Kaolin | - | 92 % |

Man erhält ein anwendungsfertiges Stäubemittel, indem der Wirkstoff mit dem Träger vermischt auf einer-geeigneten Mühle vermahlen wird.

### Beispiel F3: Extruder-Granulat

| | |
|---|---|
| Wirkstoff in Kristallmodifikation B | 10 % |
| N-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet. Ein solches Granulat ist unbegrenzt lagerfähig bei tiefen Temperaturen (-20°C bis +20°C) wie auch bei höheren Temperaturen (+20°C bis +55°C).

### Beispiel F4: Umhüllungs-Granulat

| | |
|---|---|
| Wirkstoff in Kristallmodifikation B | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man ein staubfreies Umhüllungs-Granulat. (MG = Molekulargewicht)

### Beispiel F5: Suspensions-Konzentrat

| | |
|---|---|
| Wirkstoff in Kristallmodifikation B | 40 % |
| Ethylenglykol | 10 % |
| Nonylphenolpolyethylenglykolether (15 Mol Ethylenoxid) | 6 % |
| N-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein bei tiefen wie höheren Temperaturen lagerstabiles Suspensions-Konzentrat, aus welchem durch Verdünnen mit Wasser Suspensionen jeder gewünschten Konzentration hergestellt werden können.

### Anwendungsbeispiele

### Beispiel A1: Physikochemisches Verhalten der beiden Kristallmodifikationen nach längerer Lagerung

Von jeder Kristallmodifikation A und B wird jeweils eine Formulierung gemäss Beispiel F1 c) hergestellt und ihr physikochemisches Verhalten bestimmt. Nach 6 Monaten Lagerung bei 50°C hat sich dieses Verhalten bei jener Formulierung, die die erfindungsgemässe Kristallmodifikation B enthält, nicht verändert. Die Suspendier- und Dispergierbarkeit der Formulierung, die die bekannte Kristallmodifikation A enthält, ist demgegenüber nach 6 Monaten bei 22°C bzw. nach einem Monat bei 35°C deutlich schlechter.
Es ergeben sich folgende Werte (RT = Raumtemperatur).

| | | | | | | | | | | | | | | | |
|---|---|---|---|---|---|---|---|---|---|---|---|---|---|---|---|
| Lagerdauer [Monate] | 1 | | | | | 3 | | | | | 6 | | | | |
| Lagertemperatur [°C] | -18 | RT | 35 | 40 | 50 | -18 | RT | 35 | 40 | 50 | -18 | RT | 35 | 40 | 50 |

| Modifikation A | | | | | | | | | | | | | | | |
|---|---|---|---|---|---|---|---|---|---|---|---|---|---|---|---|
| Schwebefähigkeit | + | + | /- | - | - | + | + | /- | - | - | + | + | /- | - | - |
| Siebrückstand | + | + | / | - | - | + | + | / | - | - | + | + | / | - | - |

| Modifikation B | | | | | | | | | | | | | | | |
|---|---|---|---|---|---|---|---|---|---|---|---|---|---|---|---|
| Schwebefähigkeit | + | + | + | + | + | + | + | + | + | + | + | + | + | + | + |
| Siebrückstand | + | + | + | + | + | + | + | + | + | + | + | + | + | + | + |

### Beispiel A2: Wirkung gegen Venturia inaequalis auf Apfeltrieben

Apfelstecklinge mit 10 bis 20 cm langen Frischtrieben werden mit einer aus Spritzpulver des Wirkstoffes der Kristallmodifikation B mit einem Schmelzpunkt von 73 - 75°C hergestellten Spritzbrühe (0,006 % Aktivsubstanz) besprüht Nach 24 Stunden werden die behandelten Pflanzen mit einer Konidiensuspension des Pilzes infiziert. Die Pflanzen werden dann während 5 Tagen bei 90 bis 100 % relativer Luftfeuchtigkeit inkubiert und während 10 weiteren Tagen in einem Gewächshaus bei 20 bis 24°C aufgestellt. Der Schorfbefall wird 15 Tage nach der Infektion beurteilt.

(4-Cyclopropyl-6-methyl-pyrimidin-2-yl-)-phenyl-amin der Kristallmodifikation B reduziert den *Venturia*-Befall auf 0 bis 10 %. Unbehandelte aber infizierte Kontrollpflanzen weisen dagegen einen *Venturia*-Befall von 100 % auf.

## Patentansprüche

1. (4-Cyclopropyl-6-methyl-pyrimidin-2-yl)-phenyl-amin der Kristallmodifikation B in hoher eutektischer Reinheit (Gehalt mindestens 98%) mit einem Schmelzpunkt oberhalb 73°C, bevorzugt 73 - 75°C, charakterisiert durch sein IR-Spektrum mit einer NH-Bande bei 3200-3300 cm⁻¹ und einem Röntgenpulverdiagramm aufgenommen mit einer Guinier-Kamera in Durchstrahlungsgeometrie mit Quarz als internem Standard und unter Verwendung von Kupfer-Kα₁-Strahlung (λ = 1.54060Å) mit folgenden Daten
| d-Wert(Å) | Intensität | d-Wert(Å) | Intensität |
|---|---|---|---|
| 12.9 | mittel | 3.97 | schwach |
| 8.7 | stark | 3.86 | mittel |
| 6.8 | stark | 3.80 | sehr stark |
| 6.1 | schwach | 3.78 | mittel |
| 5.93 | sehr schwach | 3.67 | mittel |
| 5.66 | stark | 3.56 | mittel |
| 5.39 | schwach | 3.54 | sehr schwach |
| 5.19 | sehr schwach | 3.42 | mittel |
| 4.96 | schwach | 3.38 | schwach |
| 4.81 | mittel | 3.30 | mittel |
| 4.75 | mittel | 3.25 | sehr schwach |
| 4.55 | sehr stark | 3.16 | schwach |
| 4.47 | mittel | 3.09 | schwach |
| 4.36 | schwach | 3.04 | sehr schwach |

2. Verfahren zur Herstellung von (4-Cyclopropyl-6-methyl-pyrimidin-2-yl-)-phenyl-amin der Kristallmodifikation B gemäss Anspruch 1, **dadurch gekennzeichnet, dass** die Kristallisation oberhalb von 26°C durchgeführt wird.

3. Verfahren gemäss Anspruch 2, **dadurch gekennzeichnet, dass** die Kristallisation in Gegenwart eines Lösungsvermittlers durchgeführt wird.

4. Verfahren gemäss Anspruch 3, **gekennzeichnet durch** Verwendung eines organischen Lösungsmittels als Lösungsvermittler.

5. Verfahren gemäss Anspruch 4, **gekennzeichnet durch** Verwendung von Toluol, Isopropanol oder Methylcyclohexan als Lösungsvermittler.

6. Verfahren zur Herstellung von (4-Cyclopropyl-6-methyl-pyrimidin-2-yl-)-phenyl-amin der Kristallmodifikation B gemäss Anspruch 1, **dadurch gekennzeichnet, dass** es durch Schmelzkristallisation gewonnen wird.

7. Verfahren gemäss Anspruch 6, **dadurch gekennzeichnet, dass** die Herstellung in einem Kratzkessel erfolgt.

8. Verfahren gemäss Anspruch 6, **dadurch gekennzeichnet, dass** die sich bildenden Kristalle von der gekühlten Kesselwand abgeschabt werden.

9. Verfahren gemäss Anspruch 8, **dadurch gekennzeichnet, dass** die Kesselwand eine Temperatur von 40°C bis 60°C hat.

10. Verfahren gemäss Anspruch 9, **dadurch gekennzeichnet, dass** die Temperatur 50°C ist.

11. Verfahren gemäss Anspruch 6, **dadurch gekennzeichnet, dass** bei der Schmelzkristallisation zuerst eine mit Kristallkeimen versehene Schmelze erzeugt wird.

12. Verfahren gemäss Anspruch 6, **dadurch gekennzeichnet, dass** zur Vervollständigung des Kristallisationsprozesses die Schmelze weiter abgekühlt wird.

13. Fungizides Mittel enthaltend als Wirkstoff (4-Cyclopropyl-6-methyl-pyrimidin-2-yl-)-phenyl-amin der Kristallmodifikation B mit einem Schmelzpunkt oberhalb.73°C in einer fungizid wirksamen Menge, zusammen mit einem geeigneten Trägermaterial.

14. Verfahren zur Herstellung eines Mittels gemäss Anspruch 13, **dadurch gekennzeichnet, dass** man (4-Cyclopropyl-6-methyl-pyrimidin-2-yl-)-phenyl-amin der Kristallmodifikation B mit einem Schmelzpunkt oberhalb 73°C mit einem geeigneten festen oder flüssigen Zusatzstoff und/oder Tensid innig vermischt.

15. Verfahren zur Herstellung eines Mittels gemäss Anspruch 14, **dadurch gekennzeichnet, dass** man eine mit Kristallkeimen von (4-Cyclopropyl-6-methylpyrimidin-2-yl-)-phenyl-amin der Kristallmodifikation B mit einem Schmelzpunkt oberhalb 73°C versehene Schmelze über eine geeignete Verteilvorrichtung auf eine gekühlte Fläche leitet, von wo aus der kristallisierte Wirkstoff vorzugsweise direkt in eine für den jeweiligen Zweck angemessene Formulierungsanlage eingeschleust wird.

## Claims

1. (4-Cyclopropyl-6-methyl-pyrimidin-2-yl)-phenyl-amine of crystal modification B of high eutectic purity (content at least 98 %) and having a melting point higher than 73°C, preferably from 73 to 75°C, which has an IR spectrum having an NH band at 3200-3300cm⁻¹ and an X-ray powder diagram, recorded using a Guinier camera in transmission geometry using quartz as internal standard and using copper-Kα₁ irradiation (λ = 1.54060Å), having the following data
| d-value (Å) | Intensity | d-value (Å) | Intensity |
|---|---|---|---|
| 12.9 | medium | 3.97 | weak |
| 8.7 | strong | 3.86 | medium |
| 6.8 | strong | 3.80 | very strong |
| 6.1 | weak | 3.78 | medium |
| 5.93 | very weak | 3.67 | medium |
| 5.66 | strong | 3.56 | medium |
| 5.39 | weak | 3.54 | very weak |
| 5.19 | very weak | 3.42 | medium |
| 4.96 | weak | 3.38 | weak |
| 4.81 | medium | 3.30 | medium |
| 4.75 | medium | 3.25 | very weak |
| 4.55 | very strong | 3.16 | weak |
| 4.47 | medium | 3.09 | weak |
| 4.36 | weak | 3.04 | very weak. |

2. A process for the preparation of (4-cyclopropyl-6-methyl-pyrimidin-2-yl)-phenyl-amine of crystal modification B according to claim 1, wherein the crystallisation is carried out at a temperature higher than 26°C.

3. A process according to claim 2, wherein the crystallisation is carried out in the presence of a solubiliser.

4. A process according to claim 3, wherein an organic solvent is used as solubiliser.

5. A process according to claim 4, wherein toluene, isopropanol or methylcyclohexane is used as solubiliser.

6. A process for the preparation of (4-cyclopropyl-6-methyl-pyrimidin-2-yl)-phenyl-amine of crystal modification B according to claim 1, wherein that compound is obtained by melt-crystallisation.

7. A process according to claim 6, wherein the preparation is carried out in a scraper kettle.

8. A process according to claim 6, wherein the crystals that form are scraped off the cooled kettle wall.

9. A process according to claim 8, wherein the kettle wall is at a temperature of from 40°C to 60°C.

10. A process according to claim 9, wherein the temperature is 50°C.

11. A process according to claim 6, wherein in the melt-crystallisation first of all a melt containing seed crystals is produced.

12. A process according to claim 6, wherein in order to complete the crystallisation process the melt is cooled further.

13. A fungicidal composition comprising as active ingredient (4-cyclopropyl-6-methylpyrimidin-2-yl)-phenyl-amine of crystal modification B having a melting point higher than 73°C in a fungicidally effective amount, together with a suitable carrier.

14. A process for the preparation of a composition according to claim 13, which comprises intimately mixing (4-cyclopropyl-6-methyl-pyrimidin-2-yl)-phenyl-amine of crystal modification B having a melting point higher than 73°C with a suitable solid or liquid adjuvant and/or surfactant.

15. A process for the preparation of a composition according to claim 14, which comprises passing a melt containing seed crystals of (4-cyclopropyl-6-methyl-pyrimidin-2-yl)-phenyl-amine of crystal modification B having a melting point higher than 73°C, using a suitable distributing apparatus, to a cooled surface, from which the crystallised active ingredient is preferably introduced directly into a formulation apparatus suitable for the particular purpose.

## Revendications

1. (4-cyclopropyle-6-méthyle-pyrimidin-2-yle)-phénylamine de modification cristalline B dotée d'une grande pureté eutectique (teneur au moins de 98 %) avec un point de fusion supérieur à 73°C, de préférence entre 73°C et 75°C, **caractérisée par** son spectre IR pourvu d'une bande NH caractéristique à 3200 - 3300 cm⁻ ¹ et un diagramme de diffraction des poudres enregistré avec une caméra Guinier en géométrie par irradiation avec le quartz pour norme interne et en utilisant un rayon cuivre Kα₁ (λ = 1,54060 Å) avec les données suivantes :
| Valeur d(Å) | Intensité | Valeur d(Å) | Intensité |
|---|---|---|---|
| 12,9 | Moyenne | 3,97 | Faible |
| 8,7 | Forte | 3,86 | Moyenne |
| 6,8 | Forte | 3,80 | Très forte |
| 6,1 | Faible | 3,78 | Moyenne |
| 5,93 | Très faible | 3,67 | Moyenne |
| 5,66 | Forte | 3,56 | Moyenne |
| 5,39 | Faible | 3,54 | Très faible |
| 5,19 | Très faible | 3,42 | Moyenne |
| 4,96 | Faible | 3,38 | Faible |
| 4,81 | Moyenne | 3,30 | Moyenne |
| 4,75 | Moyenne | 3,25 | Très faible |
| 4,55 | Très forte | 3,16 | Faible |
| 4,47 | Moyenne | 3,09 | Faible |
| 4,36 | Faible | 3,04 | Très faible |

2. Procédé de préparation de la (4-cyclopropyle-6-méthyle-pyrimidin-2-yle)-phénylamine de modification cristalline B selon la revendication 1, **caractérisé en ce que** la cristallisation est réalisée au-dessus de 26°C.

3. Procédé selon la revendication 2, **caractérisé en ce que** la cristallisation est réalisée en présence d'un agent de solubilisation.

4. Procédé selon la revendication 3, **caractérisé par** l'utilisation d'un solvant organique comme agent de solubilisation.

5. Procédé selon la revendication 2, **caractérisé par** l'utilisation du toluène, de l'isopropanol ou du méthylcyclohexane comme agent de solubilisation.

6. Procédé de préparation de la (4-cyclopropyle-6-méthyle-pyrimidin-2-yle)-phénylamine de modification cristalline B selon la revendication 1, **caractérisé en ce qu'**elle est obtenue par cristallisation par fusion.

7. Procédé selon la revendication 6, **caractérisé en ce que** la préparation s'effectue dans un caisson à racloir.

8. Procédé selon la revendication 6, **caractérisé en ce que** les cristaux se formant sont raclés de la paroi refroidie du caisson.

9. Procédé selon la revendication 8, **caractérisé en que** la paroi du caisson a une température située entre 40°C et 60°C.

10. Procédé selon la revendication 9, **caractérisé en ce que** la température se situe à 50°C.

11. Procédé selon la revendication 6, **caractérisé en ce que** l'on produit d'abord une matière fondue dotée de germes de cristaux lors de la cristallisation par fusion.

12. Procédé selon la revendication 6, **caractérisé en ce que** la matière fondue est encore refroidie pour achever le processus de cristallisation.

13. Produit fongicide contenant comme substance active la (4-cyclopropyle-6-méthyle-pyrimidin-2-yle)-phénylamine de modification cristalline B dotée d'un point de fusion supérieur à 73°C en une quantité efficace du point de vue fongicide en même temps qu'un matériau véhicule approprié.

14. Procédé de préparation d'un produit selon la revendication 13, **caractérisé en ce que** l'on mélange intimement la (4-cyclopropyle-6-méthyle-pyrimidin-2-yle)-phénylamine de modification cristalline B dotée d'un point de fusion supérieur à 73°C avec un additif et/ou un tensioactif solide ou liquide approprié.

15. Procédé de préparation d'un produit selon la revendication 14, **caractérisé en ce que** l'on dirige une matière fondue dotée de germes de cristaux de (4-cyclopropyle-6-méthyle-pyrimidin-2-yle)-phénylamine de modification cristalline B dotée d'un point de fusion supérieur à 73°C sur une surface refroidie, à partir de laquelle on introduit la substance active cristallisée de préférence directement dans un appareil de formulation adapté aux destinations respectives.
